# EUROPEAN PATENT APPLICATION

(11) **EP 4 686 472 A1**
(43) Date of publication of application: **04.02.2026**
(21) Application number: 24192529.6
(22) Date of filing: 02.08.2024
(51) Int. Cl.: A61K 31/18, A61K 31/255, A61K 31/34, A61K 31/426, A61K 31/433, A61K 31/496, A61K 31/506, A61K 31/549, A61K 31/573, A61K 31/69, A61K 38/00, A61K 39/00, A61P 27/02

(54) **CXCL8 INHIBITORS FOR USE IN THE TREATMENT OF OCULAR GRAFT-VERSUS-HOST DISEASE**

(71) Applicant: Dompé farmaceutici SpA, 20122 Milano (IT)
(72) Inventor: MANTELLI, Flavio, 67100 L'Aquila (IT); SACCHETTI, Marta, 80161 L'Aquila (IT); ARAMINI, Andrea, 67100 L'Aquila (IT)
(74) Representative: Dompé farmaceutici Spa

(57) **Abstract**

The invention relates to CXCL8 inhibitors for use in the treatment of ocular graft-versus-host disease.

## Description

### TECHNICAL FIELD

The invention relates to CXCL8 inhibitors for use in the treatment of ocular graft-versus-host disease (oGVHD).

### BACKGROUND OF THE INVENTION

Allogenic hematopoietic stem cell transplantation (AHSCT) is an established treatment of many haemato-oncological, immunological and hereditary conditions (Balassa et al., British Journal of Hospital Medicine 2019 80:1,33-39). In AHSCT, the stem cells are derived from a human leukocyte antigen (HLA)- matched donor rather than from the patient's own tissue.

One of the major complications occurring after AHSCT is graft-versus-host disease (GVHD). GVHD presents as a destruction of normal host tissues due to an exacerbated systemic inflammatory response. GVHD is the most common cause of non-relapsing morbidity and mortality in patients greater than 2 years from transplant, occurring in 30% to 70% of patients (Arai et al., Blood 2011 Oct 13;118(15):4242-9; Zeiser R et al., N Engl J Med 2017;377:2565-79).

GVHD is classified either as acute or chronic. In the past, the classification was based on the time elapsed between the transplant and the occurrence of symptoms, so that GVHD was defined as "acute" when the symptoms occurred within 100 days of transplant and "chronic" when the symptoms occurred thereafter (Balasubramaniam SC et al., Eye Contact Lens 2015;41:256-61 ; Lee SJ et al., Blood 2017;129:30-7). Nowadays, the classification is based primarily on the specific tissue involvement and clinical manifestation of the subject.

Specifically, acute GVHD affects the skin, oral and gastrointestinal mucous membranes, lungs, liver (Balasubramaniam SC et al., Eye Contact Lens 2015;41:256-61), and eyes (Saito T et al., Int J Hematol 2002;75:332-4). Chronic GVHD more frequently involves the eyes, musculoskeletal systems, lymphohematopoietic systems, hair and nails, and genitals, as well as the other areas affected in acute GVHD (Balasubramaniam SC et al., Eye Contact Lens 2015;41:256-61; Zeiser R et al., N Engl J Med 2017;377:2565-79). Ocular manifestations and symptoms of GVHD are known under the name of "ocular GVHD" (oGVHD) and are found both in acute and chronic GVHD. Approximately 40% to 90% of patients with chronic GVHD develop ocular symptoms (Arai et al., Blood 2011 Oct 13;118(15):4242-9).

Acute oGVHD commonly presents as pseudomembranous or hemorrhagic conjunctivitis and its main symptoms are eye pain and lacrimation. Corneal signs include epithelial sloughing, corneal epithelial keratitis, or filamentary keratitis which may be secondary to conjunctival cicatrization due to the disease. Some patients may present with lagophthalmos (Saito T et al., Int J Hematol 2002;75:332-4; Janin A et al., Hum Pathol 1996;27:307-9; Uchino M et al., Acta Ophthalmol Scand 2006;84:545-8; Nassar A et al., Saudi J Ophthalmol 2013;27:215-22; Sung AD et al., Stem Cells Transl Med 2013;2:25-32; Nair et al., Indian J ophthalmol 2021;69:1038-50). Based on the clinical manifestation and on the severity of symptoms, acute oGVHD is classified as stage 1, stage 2, stage 3 or stage 4 (Jabs DA et al., Arch Ophthalmol 1989;107:1343-8).

Patients affected by oGVHD usually complain of relevant dry eye symptoms, such as chronic redness, eye pain, photophobia, foreign body sensation, and decreased vision. These clinical manifestations are the result of changes in the ocular surface, particularly in the cornea, conjunctiva, lacrimal glands, Meibomian Glands, and eyelids (Jabs DA et al., Arch Ophthalmol 1989;107:1343-8; Balaram M et al., Ocul Surf 2005; 3:203-11; Mohammadpour M, Cornea 2007;26:225-6; Hwang HS et al., Ocul Surf 2019;17:464-9; Que L et al., Eye Contact Lens 2018;44(Suppl 2):S169-75; Ogawa Y et al., Invest Ophthalmol Vis Sci 2003;44:1888-96; Giannaccare G et al., Ocul Surf 2019;17:98-103). Other signs which may be found in chronic oGVHD include cataract, episcleritis, scleritis, posterior scleritis, anterior uveitis, vitritis, and serious choroidal detachment (Dietrich-Ntoukas T et al., Cornea 2012;31:299-310; Nair S et al., Indian J Ophthalmol 2021;69:1038-50). Different criteria are known for diagnosing and scoring chronic oGVHD (Jagasia MH et al., Biol Blood Marrow Transplant 2015;21:389-401 e1; Robinson MR et al., Bone Marrow Transplant 2004;33:1031-5).

Ocular GVHD can be debilitating and severely impact patients' quality of life. Currently there are no widely accepted guidelines available for its management, and no suggested regime of treatment that is completely satisfactory.

This condition is currently managed with localized treatments including topical lubricants such as preservative-free artificial tears, autologous serum tears, topical immunosuppressive treatments and topical anti-inflammatories. These include topical corticosteroids, topical tacrolimus and topical cyclosporine A, but these treatments are not specifically approved by Regulatory Authorities for oGVHD (Robinson et al., Bone Marrow Transplant 2004, May;33(10):1031 - 5; Malta JB et al., Cornea 2010;29:1392-6; Couriel D. et al. Biology of Blood and Marrow Transplantation. 2006; 12(4):375-396; Nassiri N et al., Journal of Ophthalmic and Vision Research. 2013; 8(4): 351-358; Nassar A et al., Saudi Journal of Ophthalmology. 2013; 27 (3): 215-222; Giannaccare G et al., Graefes Arch Clin Exp Ophthalmol 2019 Ju!;257(7):1341-1351). Nevertheless, in spite of the use of the above-mentioned therapies, the management of oGVHD is still not satisfactory due to the following reasons. First, the above-mentioned treatments are associated with relevant drawbacks. For instance, it has been observed that the efficacy of topical corticosteroids is reduced in patients with Dry Eye Disease (DED) secondary to GVHD compared to those with conventional DED, even when controlling for clinical disease severity (Yin J et al., 2018, Am J Ophthalmol 190:17-23). In addition, the use of topical corticosteroids is strongly limited by the potential long-term adverse effects, including raised intraocular pressure (leading to Glaucoma), cataract formation, decreased wound healing, and predisposition to infection, all fearsome non-GVHD ocular complications of hematological patients (Inamoto Y et al., Bone Marrow Transplant, 2019 May;54(5):648-661). Furthermore, the management of oGVHD is challenging due to the recurrent course and refractory nature of this pathology to the treatments indicated above (Nair et al., Indian J ophthalmol 2021 ;69:1038-50; Greenan et al., BMJ Case Rep. 2019; 12(12): e232579).

Thus, there is still a high need to develop safe, targeted therapies which are effective against oGVHD.

CXCL8 (interleukin-8, IL-8) is an endogenous chemotactic factor produced by most nucleated cells such as fibroblasts, macrophages, endothelial and epithelial cells.

As reported, the biological activity of CXCL8 is mediated by the interaction of CXCL8 with CXCR1 and CXCR2 membrane receptors belonging to the family of seven transmembrane receptors and expressed on the surface of human neutrophils and of several types of T-cells (L. Xu et al., J. Leukocyte Biol., 57, 335, 1995). Although CXCR1 activation is known to play a crucial role in CXCL8-mediated chemotaxis, it has been supposed that also CXCR2 activation could play a pathophysiological role in chronic inflammatory diseases.

Different CXCL8 inhibitors have been developed and are well known to the skilled person.

WO2000/024710 discloses N-(2-aryl-propionyl)-sulfonamides, having inhibitory activity of neutrophils chemotaxis and degranulation induced by interleukin-8, and their use in the prevention and treatment of tissue damage due to the exacerbate recruitment of polymorphonuclear neutrophils (leukocytes PMN) at the inflammatory sites, in particular in the treatment of psoriasis, rheumatoid arthritis, ulcerative colitis, acute respiratory insufficiency, idiopathic fibrosis, glomerulonephritis.

WO2005/090295 discloses (R)-2-[4-(trifluoromethanesulfonyloxy)phenyl]propionic acid derivatives, which are used as inhibitors of the chemotaxis of polymorphonucleate and mononucleate cells, particularly in the treatment of neutrophils-dependent pathologies. The use of said compounds in the treatment of psoriasis, ulcerative colitis, melanoma, angiogenesis, chronic obstructive pulmonary disease (COPD), bullous pemphigo, rheumatoid arthritis, idiopathic fibrosis, glomerulonephritis and in the prevention and treatment of damages caused by ischemia and reperfusion is also disclosed.

WO2010/031835 discloses 2-aryl-propionic acids and derivatives, substituted in the 4 position by 2-amino-heterocycles as CXCL8-induced chemotaxis inhibitors, useful in the prevention and treatment of tissue damage due to the exacerbated recruitment of polymorphonucleated neutrophils (PMN leukocytes) at inflammation sites. The use of said compounds in the treatment of transient cerebral ischemia, damages caused by ischemia and reperfusion, bullous pemphigo, rheumatoid arthritis, idiopathic fibrosis and glomerulonephritis is also disclosed.

The present invention is aimed at providing an effective treatment of ocular graft-versus-host disease (oGVHD).

### SUMMARY OF THE INVENTION

The invention is directed to a CXCL8 inhibitor for use in treatment of ocular graft-versus-host disease (oGVHD) in a subject.

The invention is also directed to an ophthalmic composition comprising a CXCL8 inhibitor and at least one ophthalmologically acceptable excipient or carrier for use in the treatment of ocular graft versus host disease (oGVHD) in a subject.

The invention is also directed to a method of treating ocular graft versus host disease (oGVHD) in a subject, which comprises administering an effective amount of one or more CXCL8 inhibitors to a subject in need thereof.

The invention is also directed to the use of a CXCL8 inhibitor in the manufacture of a medicament for the treatment of ocular graft versus host disease (oGVHD) in a subject.

### BRIEF DESCRIPTION OF FIGURES

**Figure 1** shows the timeline of the study described in Example 1 below. To assess the baseline of all the animals, F+L pictures and GVHD score were assessed before the induction. The GVHD model was induced at day 0 and on day 1 animals were injected with BM/BM+T cells. The treatment was applied twice per day from day 7 to day 29. On days 2, 7, 21, and 29 F+L pictures were taken and the GVHD score was quantified. Moreover, the FRT was performed before the sacrifice, which occurred on day 29.
**Figure 2** shows in panel A) GVHD model induction as demonstrated by the milder GVHD score measured in the BM group when compared to the BM+T group, and in panel B) GVHD score progression from day 0 to 29. Statistical analysis by t-test and two-way ANOVA, followed by Tukey's test. *p<0.5, ****p<0.0001.
**Figure 3** shows in panel A) representative pictures of fluorescein staining of the cornea in different groups at day 7 and 29, and in panel B) graphs showing fluorescein staining progression of all groups from day 7 to 29. Statistical analysis by two-way ANOVA, followed by Tukey's test. *p<0.5, **p<0.01.
**Figure 4** shows opacity score progression of all groups from day 7 to 29. Statistical analysis by two-way ANOVA, followed by Tukey's test.
**Figure 5** shows the eyelid score progression from day 7 to 29. Statistical analysis by two-way ANOVA, followed by Tukey's test. *p<0.5.
**Figure 6** shows the number of CD45⁺ cell/field reported as a column bar graph. Statistical analysis by one-way ANOVA. Number of samples used for the analysis: 26.

### DETAILED DESCRIPTION OF THE INVENTION

It has been surprisingly found that inhibitors of CXCL8 are effective in the treatment of ocular graft-versus-host disease (oGVHD).

Accordingly, the invention is directed to a CXCL8 inhibitor for use in the treatment of ocular graft-versus-host disease (oGVHD) in a subject.

The term "treatment" as used herein refers to:
- the eradication or amelioration of the disorder being treated or of one or more of the symptoms associated thereof, notwithstanding the fact that the patient may still be afflicted with the underlying disorder. Preferably, said one or more symptoms are selected from dry eye, foreign body sensation, redness, epiphora, photophobia, blurred vision and eye irritation and ocular discomfort;
- the reduction or inhibition of complications or chronicization of the disorder being treated, notwithstanding the fact that the patient may still be afflicted with the underlying disorder. Preferably, said complications are selected from meibomian gland obstruction, anterior and posterior blepharitis, scarring of the lacrimal gland leading to decreased tear production, conjunctival hyperemia with pseudomembrane and membrane formation, conjunctival necrosis, cicatricial scarring and fibrosis, punctate epithelial keratopathy, filamentary keratitis, corneal erosions, thinning, ulcerations and perforations.

According to a preferred embodiment, said subject is a human subject.

According to a preferred embodiment, said CXCL8 inhibitor is for use in the treatment of ocular inflammation associated with ocular graft-versus-host disease.

According to a preferred embodiment, also in combination with any of the above embodiments, said treatment stabilizes corneal epitheliopathy.

According to a preferred embodiment, also in combination with any of the above embodiments, said treatment improves the eyelid score.

According to a preferred embodiment, also in combination with any of the above embodiments, said treatment reduces leukocyte infiltration.

According to an embodiment, said ocular graft-versus-host disease is chronic ocular graft-versus-host disease.

According to an alternative embodiment, said ocular graft-versus-host disease is acute ocular graft-versus-host disease.

The term "CXCL8 inhibitors" in accordance with the present invention means any compound able to inhibit the biological activity of CXCL8.

Preferably, the CXCL8 inhibitor for use according to the present invention is an anti-CXCL8 antibody or a CXCL8 receptor(s) inhibitor.

Preferably said CXCL8 receptor(s) inhibitor is a CXCR1 inhibitor, which inhibits the activity of CXCL8 mediated by the CXCR1 receptor, or a CXCR1/CXCR2 inhibitor, which inhibits the activity of CXCL8 mediated by both the CXCR1 and CXCR2 receptors.

Said CXCL8 receptor(s) inhibitor preferably inhibits the binding of CXCL8 to the CXCR1 receptor (CXCR1 receptor inhibitor) or to both the CXCR1 and CXCR2 receptors (dual CXCR1 and CXCR2 receptors inhibitor), or prevents or blocks the intracellular signaling activated by the binding of CXCL8 to the CXCR1 receptor (CXCR1 receptor inhibitor) or to both the CXCR1 and CXCR2 receptors (dual CXCR1 and CXCR2 receptors inhibitor).

Methods and tests for determining the inhibition of the biological activity of CXCL8 and/or of CXCR1/2 receptors and for classifying a compound as "CXCL8 inhibitor", "CXCR1 inhibitor" or as "CXCR1/CXCR2 inhibitor" are known in the art and are described, for example, in Moriconi et al., J. Med. Chem. 2007, 50, 3984-4002, and in Brandolini et al., Scientific Reports (2019) 9:11729.

According to a preferred embodiment, the CXCL8 receptor(s) inhibitor is an antagonist of the CXCR1 receptor or an antagonist of both CXCR1 and CXCR2 receptors.

According to another preferred embodiment, the CXCL8 receptor(s) inhibitor is an allosteric inhibitor or an orthosteric antagonist of CXCR1 receptor or of both CXCR1 and CXCR2 receptors.

Alternatively, the CXCL8 inhibitor preferably binds to CXCL8, thus preventing its binding to its receptors.

Said CXCL8 receptor(s) inhibitor is preferably able to inhibit in an in-vitro assay at least 60%, preferably at least 70%, more preferably at least 80%, even more preferably at least 90% of PMNs chemotaxis induced by 1 nM CXCL8 at a concentration equal or below 500 nM, preferably below 100 nM.

More preferably, the CXCL8 receptor(s) inhibitor according to the invention has an IC₅₀ value towards CXCR1 receptor in the low nanomolar range, preferably lower than 10 nanomolar, more preferably in the range 0.02-5 nanomolar.

Preferably, the CXCL8 inhibitor for use according to the invention is selected from small molecules, peptides and antibodies, more preferably it is a small molecule.

The term "small molecule" refers to an organic compound having a molecular weight of 900 Daltons or lower.

CXCL8 inhibitors, in particular CXCL8 receptor(s) inhibitors, as defined above, are well known in the art.

To date, several CXCL8 inhibitors, such as small molecules, peptides and antibodies, have been disclosed, many of which are currently undergoing clinical trials or are used in therapy (Jie Jack, Expert Opinion Ther. Patents, 2001, 11(12), Chao J. et al., Bioorganic & Medicinal Chemistry Letters 17, 2007, p. 3778-3783, Busch-Petersen J. Current Topics in Medicinal Chemistry, 2006, 6, p. 1345-135, Allegretti et al, Immunology Letters 2012, Vol. 145, p. 68-78, Sitaru et al., Internal and Emergency Medicine (2023) 18: 1647-1664). Preferably, the CXCL8 inhibitor for use according to the invention is selected from the group comprising, preferably consisting of:
- the anti-CXCL-8 antibodies ABCream, BMS-986253 and ABX-IL-8;
- RP-72, PAC-G-31-P, SCH-N,
- Navarixin, having formula: 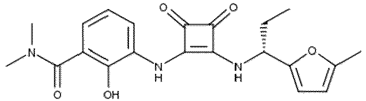
- SX-517, having formula: 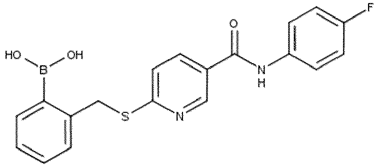
- SX-576, having formula: 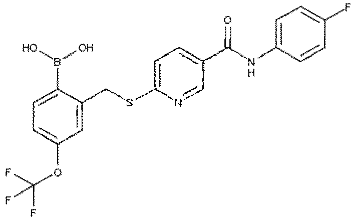
- SX-682, having formula: 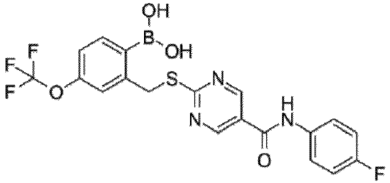
- compounds having formula: 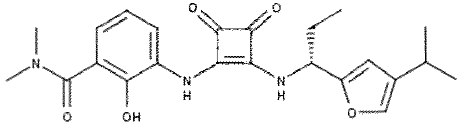 or 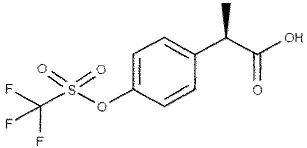 or 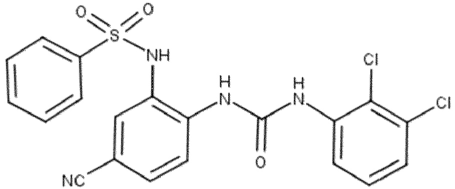 and
- 5-[3-(2-Fluorophenyl)ureido]-1-(2-hydroxypropyl)-1H-pyrazole-4-carboxylic acid ethyl ester
- 5-[3-(3-Fluorophenyl)ureido]-1-(2-hydroxypropyl)-1H-pyrazole-4-carboxylic acid ethyl ester
- 3-[2-[1(R)-(4-Bromofuran-2-yl)propylamino]-3,4-dioxo-1-cyclobutenylamino]-2-hydroxy-N,N-dimethylbenzamide
- 3-[2-[1(R)-(4-Chlorofuran-2-yl)propylamino]-3,4-dioxo-1-cyclobutenylamino]-2-hydroxy-N,N-dimethylbenzamide
- Trifluoromethanesulfonic acid 4-[1(R)-(N-isopropylcarbamoyl)ethyl]phenyl ester
- 2-Hydroxy-3-[4-[1(R)-(4-isopropylfuran-2-yl)propylamino]-1 -oxo-1,2,5-thiadiazol-3-ylamino]-N,N-dimethylbenzamide
- 3-(2-Chlorophenylamino)-7-nitro-4H-1,2,4-benzothiadiazin-5-ol 1,1-dioxide
- 1-[3-[4-[3-(4-Fluorophenyl)isoxazol-5-yl]phenoxy]propyl]-4-methylpiperazine
- N-(2-[(2,3-Difluorobenzyl)sulfanyl]-6-[[(2R,3S)-3,4-dihydroxybutan-2-yl]oxy]pyrimidin-4-yl)azetidine-1-sulfonamide; and
- the compounds of formula (I) and (II) described hereinbelow.

According to a preferred embodiment, said CXCL8 inhibitor has general formula (I): 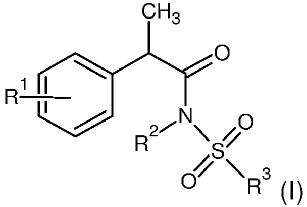 wherein
R¹ is selected from a linear or branched C₁-C₆ alkyl, benzoyl, phenoxy, and trifluoromethanesulfonyloxy;
R² is selected from hydrogen and a linear or branched C₁-C₃ alkyl; and
R³ is a linear or branched C₁-C₆ alkyl or trifluoromethyl,
or a pharmaceutically acceptable salt thereof.

According to the present invention, "C₁-C₆-alkyl" represents a linear or branched alkyl chain containing 1 to 6 carbon atoms.
R¹ is preferably selected from benzoyl, isobutyl, and trifluoromethanesulfonyloxy. R¹ is preferably linked to the phenyl ring in 3- or 4-position. According to the most preferred embodiment, R¹ is 3-benzoyl, 4-isobutyl or 4-trifluoromethanesulfonyloxy.
R² is preferably selected from hydrogen and methyl.
R³ is preferably selected from linear or branched C₁-C₆ alkyl, more preferably from linear of branched C₁-C₃ alkyl. According to the most preferred embodiment, R³ is methyl.

The chiral carbon of the compounds of formula (I) is in the RS or R configuration, more preferably it is in the R configuration.

Particularly preferred compounds of formula (I) according to the invention are selected from:
- 2-(4-isobutylphenyl)propionyl methansulfonamide, preferably R-(-)-2-(4-isobutylphenyl)propionyl methansulfonamide (also known as reparixin) and pharmaceutically acceptable salts thereof, preferably the lysine salt thereof, and
- 2-[(4-trifluoromethanesulfonyloxy)phenyl]-N-methanesulfonyl propionamide, preferably R(-)-2-[(4-trifluoromethanesulfonyloxy)phenyl]-N-methanesulfonyl propionamide and pharmaceutically acceptable salts thereof, in particular the sodium salt thereof (also known as ladarixin or DF2156A).

Compounds of formula (I) are described in WO2000/024710A1 and WO2005/090295A2, which also disclose their method of synthesis. According to a further preferred embodiment, said CXCL8 inhibitor has general formula (II): 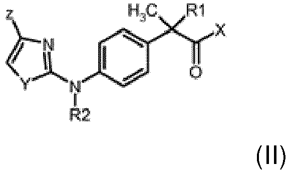 wherein:
R1 is hydrogen or linear or branched C₁-C₄ alkyl, preferably CH₃;
X is OH;
R2 is hydrogen or linear C₁-C₄ alkyl,
Y is a heteroatom selected from S, O and N,
Z is selected from linear or branched C₁-C₄ alkyl, linear or branched C₁-C₄ alkoxy, halo C₁-C₃ alkyl and halo C₁-C₃ alkoxy,
or pharmaceutically acceptable salts thereof.

Preferably, the chiral carbon of the compounds of formula (II) is in the R or S configuration, more preferably it is in the S configuration.

A particularly preferred compound of formula (II) according to the invention is 2-(4-{[4-(trifluoromethyl)-1,3-thiazol-2-yl]amino}phenyl)propanoic acid, preferably (2S)-2-(4-{[4-(trifluoromethyl)-1,3-thiazol-2-yl] amino} phenyl) propanoic acid or the sodium salt thereof.

Another particularly preferred compound of formula (II) according to the invention is 2-methyl-2-(4-{[4-(trifluoromethyl)-1,3-thiazol-2-yl]amino} phenyl)propanoic acid (DF2726Y) or a pharmaceutically acceptable salt thereof, preferably the sodium salt (DF2726A).

Compounds of formula (II) are described in WO2010/031835A2, which also discloses their method of synthesis.

Preferred CXCL8 inhibitors for use according to the invention are ladarixin or reparixin, more preferably the CXCL8 inhibitor is ladarixin.

The CXCL8 inhibitor compounds of the present invention may form stable pharmaceutically acceptable acid or base salts with an ophthalmically acceptable organic or inorganic acid or base, and in such cases administration of a compound as a salt may be appropriate.

Examples of acid addition salts may include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate.

Examples of base addition salts may include ammonium salts; alkali metal salts such as sodium, lithium and potassium salts; alkaline earth metal salts such as aluminum, calcium and magnesium salts; salts with organic bases such as dicyclohexylamine salts and N-methyl-D-glucamine; and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others. Non-toxic physiologically-acceptable salts are preferred, although other salts may be useful, such as in isolating or purifying the product. The salts may be formed by conventional means, such as by reacting the free form of the product with one or more equivalents of the appropriate acid or base in a solvent or medium in which the salt is insoluble, such as for example water or ethanol, which is removed under vacuum or by freeze drying. Certain CXCL8 inhibitor compounds may exist in tautomeric forms, and this invention includes all such tautomeric forms of those compounds unless otherwise specified.

Unless otherwise stated, structures depicted herein are also meant to include all stereochemical forms of the structure; i.e., the R and S configurations for each asymmetric centre. Thus, single stereochemical isomers as well as enantiomeric and diastereomeric mixtures of the present compounds are within the scope of the invention. Thus, this invention encompasses each diastereomer or enantiomer substantially free of other isomers (>90%, and preferably >95%, free from other stereoisomers on a molar basis) as well as a mixture of such isomers.

Particular optical isomers can be obtained by resolution of the racemic mixtures according to conventional processes, e.g., by formation of diastereomeric salts, by treatment with an optically active acid or base. Examples of appropriate acids are tartaric, diacetyltartaric, dibenzoyltartaric, ditoluoyltartaric, and camphorsulfonic acid and then separation of the mixture of diastereomers by crystallization followed by liberation of the optically active bases from these salts. A different process for separation of optical isomers involves the use of a chiral chromatography column optimally chosen to maximize the separation of the enantiomers. Still another method involves synthesis of covalent diastereomers by reacting compounds of the invention with an optically pure acid in an activated form or an optically pure isocyanate. The synthesized diastereomers can be separated by conventional means such as chromatography, distillation, crystallization or sublimation, and then hydrolysed to deliver the enantiomerically pure compound. Optically active compounds of the invention can be obtained by using active starting materials. These isomers may be in the form of a free acid, a free base, an ester or a salt.

According to an embodiment, the CXCL8 inhibitor for use according to the present invention is administered in combination with one or more of the following agents: ocular lubricants, autologous serum eye drops and other blood-derivative topical ophthalmic treatments, at least one other active ingredient for a concomitant ocular condition, including other topical anti-inflammatory and immunosuppressive drugs.

It is understood that the CXCL8 inhibitor can be administered before, along with or after the above-mentioned agent(s).

It is understood that the CXCL8 inhibitor can be selected from any of the above-described CXCL8 inhibitors.

According to a preferred embodiment, the CXCL8 inhibitor for use according to the present invention is administered topically to the eye of a subject, more preferably topically to the surface of the eye of a subject.

Preferably, the CXCL8 inhibitor for use according to the present invention is administered topically to the surface of the eye of a subject in form of eyedrops. Preferably, the CXCL8 inhibitor for use according to the present invention is administered topically to the surface of the eye of a subject in form of eyedrops, wherein the weight/volume concentration of the CXCL8 inhibitor in said eyedrops is between 0.05% and 1.5%, preferably between 0.1% and 1%, more preferably between 0.1% and 0.5%, even more preferably it is selected from 0.1% and 0.5%.

Preferably, the CXCL8 inhibitor for use according to the present invention is administered twice a day or three times a day, more preferably the CXCL8 inhibitor for use according to the present invention is administered three times a day.

Preferably, the CXCL8 inhibitor for use according to the present invention is administered topically to the surface of the eye of a subject in form of eyedrops, wherein the weight/volume concentration of the CXCL8 inhibitor in said eyedrops is selected from 0.1% and 0.5%, and wherein said CXCL8 inhibitor is administered three times a day.

It is understood that, according to any of the above-described embodiments, said CXCL8 inhibitor may be selected from any of the above-described CXCL8 inhibitors. More preferably, said CXCL8 inhibitor is selected from reparixin and ladarixin, even more preferably said CXCL8 inhibitor is ladarixin.

According to a preferred embodiment, said subject has undergone allogenic hematopoietic stem cell transplantation.

Preferably, said CXCL8 inhibitor is administered to said subject after allogenic hematopoietic stem cell transplantation starting immediately after the first signs and/or symptoms of ocular involvement indicative of an oGVHD reaction. As will be discussed in the experimental section, the present inventors have shown that topical administration of a CXCL8 inhibitor to the surface of the eye results in amelioration of oGVHD. In particular, the inventors have found that, in a mouse model of oGVHD, the topical administration of a CXCL8 inhibitor results in an improvement of corneal epitheliopathy and eyelid score, and in a reduction of leukocyte infiltration.

The invention is also directed to an ophthalmic composition comprising a CXCL8 inhibitor, as described above, and at least one ophthalmologically acceptable excipient or carrier for use in the treatment of ocular graft-versus-host disease (oGVHD) in a subject.

An "ophthalmologically acceptable excipient" is an excipient which allows delivery of a medicament to the eye and/or eyelids, to treat an ocular disease or condition without deleterious effects on the eye. Exemplary ophthalmologically acceptable excipients suitable to be included in the ophthalmic composition for use according to the present invention are listed below.

According to an embodiment, said ophthalmic composition is a liquid, eye drop composition for topical administration to the anterior segment of the eye. Said liquid composition may be in form of a solution, emulsion, or suspension. Said liquid composition may include micelles.

In one embodiment, the liquid composition is an aqueous composition. Preferably, the liquid composition is an aqueous eye drop composition.

Preferably, said liquid composition comprises ophthalmologically acceptable excipients selected from ophthalmologically acceptable viscosity enhancers, penetration enhancers, buffering agents, osmolarity regulators, preservatives and surfactants.

Viscosity enhancers have the function to increase viscosity of the composition and to improve its retention in the conjunctival sac and are preferably selected from cellulose derivatives, preferably hydroxymethylcellulose, hydroxyethylcellulose, hydroxypropylmethylcellulose, methylcellulose; polyvinylpyrrolidone and gelling agents, preferably gellan, xanthan gum and carbopol-974.

Penetration enhancers have the function of enhancing drug permeability across ocular membranes and are preferably selected from cyclodextrins, chelating agents, crown ethers, bile acids and bile salts.

Buffering agents have the function of providing and maintaining the correct pH of the formulation to be compatible for use in the eye, preferably at a pH comprised between 6 and 8. The preferred buffer is phosphate buffer, but other buffers capable of maintaining the pH within the desired range, especially buffers suitable for ophthalmic use, are also included.

Osmolarity regulators are salts able to make the liquid composition isotonic with ocular fluids. The preferred salt is sodium chloride (NaCl) but other biologically acceptable salts may be used, such as for instance potassium chloride (KCI), calcium chloride (CaCl₂) and magnesium chloride (MgCl₂) and their admixtures.

Preservatives inhibit microbial activity. Suitable preservatives include for instance quaternary ammonium compounds such as benzalkonium chloride, cetyltrimethylammonium bromide and cetylpyridinium chloride.

Surfactants have the function of making the composition stable and are preferably selected from polysorbates such as Tween 80, poloxamers such as Pluronics F68 or proteins such as serum albumin.

Said liquid, eye drop composition can be part of a kit comprising the composition, a container for holding the composition and a drop dispenser.

A "therapeutically effective amount" according to the present invention means an amount sufficient to achieve treatment of the disease.

A "prophylactically effective amount" according to the present invention means an amount sufficient to achieve prevention of the disease.

Determination of the therapeutically or prophylactically effective amounts is well within the capability of those skilled in the art based upon the achievement of a desired effect. An effective amount will depend on factors including, but not limited to, the weight of a subject and/or the degree of the disease or unwanted condition from which a subject suffers.

The invention relates also to the use of the CXCL8 inhibitor as above defined in the manufacture of a medicament for the treatment of ocular graft versus host disease (oGVHD).

The invention is also directed to a method of treating ocular graft versus host disease (oGVHD), which comprises administering an effective amount of one or more CXCL8 inhibitors as above defined to a subject in need thereof.

The invention is further illustrated by the following examples, which do not limit the scope of the invention as defined in the claims.

### EXPERIMENTAL SECTION

### EXAMPLE 1

A study was designed to evaluate the effect of topical administration of the CXCR1/2 inhibitor ladarixin (LAD) in a pre-clinical model of ocular graft-versus-host disease (oGVHD) previously described in Lasagni Vitar et al., Experimental Eye Research, Volume 212, 2021, 108825. Ocular GVHD is a post-transplant disorder that results from immune-mediated attack of recipient tissue by donor T cells contained in the transplant.

To reach this endpoint, the following clinical and biological parameters that are typically assessed to quantify oGVHD involvement have been evaluated: fluorescein staining score (indicating the grade of corneal and conjunctival epithelial damage), corneal opacity score (indicative of corneal oedema or scarring), eyelid score, and markers of inflammation.

### Materials and methods

The study was carried out in an established mouse model of ocular GVHD (Lasagni Vitar et al., Experimental Eye Research, Volume 212, 2021, 108825). At day 0, a total body irradiation using 9cGy was performed in two sessions of 4.5cGy. The subsequent day, bone marrow cells (BM) and T-cells were isolated from the bone marrow and the spleen, respectively. On the same day, animals were evenly divided in the following five groups:
1. Positive Control injected with Bone Marrow cells (BM) (n=8)
2. Negative control injected with BM together with T cells (BM+T) (n=8)
3. BM+T treated with ladarixin 1% (LAD) (n=8)
4. BM+T treated with dexamethasone 2 mg/mL (DM) (n=8)
5. BM+T treated with saline solution (VEH) (n=8)

The animals of group 1 (BM) received an injection of 10 x 10⁶ BM in the caudal vein. The animals of other groups received an injection of 10 x 10⁶ BM mixed with 2 × 10⁶ splenocytes in the caudal vein, consisting of 0.282 × 10⁶ CD4+ cells and 0.242 × 10⁶ CD8⁺ cells. All the cells were suspended in 100 µL of final injected volume.

The treatment started 7 days after irradiation and continued until day 29. Each treatment consisted of the topical application of 10 µl eyedrops twice per day of the compound assigned to each group.

Fluorescein and light pictures (F+L) were taken at day 0, 2, 7, 21 and 29 to assess corneal epitheliopathy (fluorescent pictures), the corneal opacity and the eyelid score (light pictures). Corneas were examined and photographs were taken under the slit-lamp microscope. In vivo corneal fluorescein staining was used to evaluate epithelial corneal damage. The procedure was performed as follows: a drop of 2 µL 0.1% fluorescein was administered in both the eyes of the animal. After 30 s, the excess of fluorescein was gently absorbed. Corneal epitheliopathy was evaluated with fluorescein staining and expressed as a percentage before and after treatment. Eyelid score, as a proxy for blepharitis, was assessed in 4 eyelid areas: superior eyelid, inferior eyelid, medial and lateral canthus. The score was given based on fur loss severity. For each point, a score was given from 0 to 2 (0 = no fur loss, 1 = less than 50% fur loss and 2 = more than 50% fur loss). The final eyelid score resulted from the sum of each point.

At the same time points, GVHD score was measured to evaluate the grade and severity of the disease considering the following parameters: weight loss, posture, activity, fur texture and skin integrity (Ferrari et al., Invest. Ophthalmol. Vis. Sci. 2013;54(3):1680-1688). Each parameter had a scoring range from 0 to 2 points, where 2 represents the highest severity, as represented in Table 1 below.

### GVHD Clinical Scoring System

**Table 1 - GVHD clinical scoring system. Clinical parameters adopted for systemic GVHD are shown.**

| Grade | Weight Loss | Posture | Activity | Fur Texture | Skin Integrity |
|---|---|---|---|---|---|
| 0 | < 5 % | not hunched | spontaneous activity | normal | normal |
| 1 | 5-10 % | moderare hunched | spontaneous activity reduced | ruffled | inflamed |
| 2 | > 10 % | severe hunched | severe decrease (no movement) | fur loss | ulcerate |

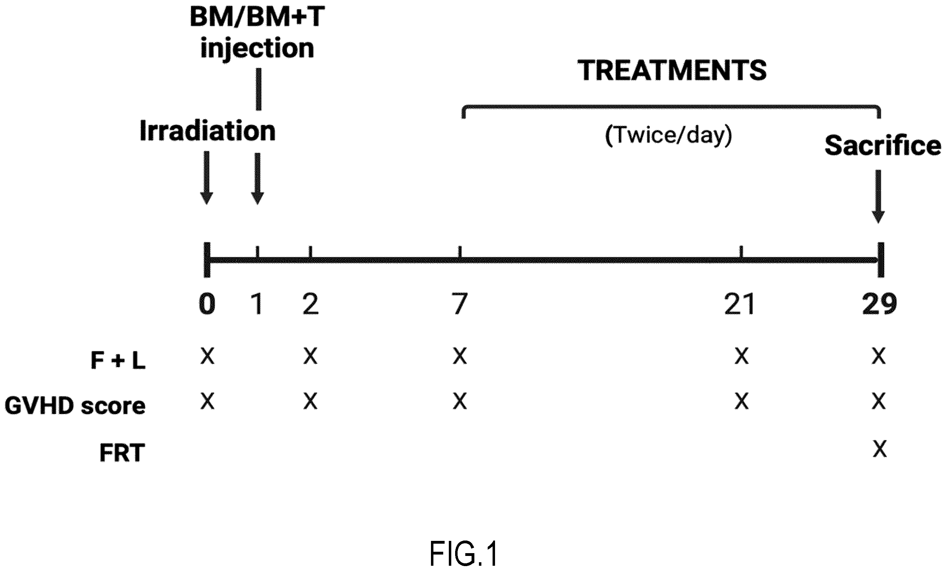

Finally, before the sacrifice, the Phenol Red Thread test (FRT) was performed to evaluate tear production (Figure 1). Briefly, the threads were held and placed in the lateral canthus of the conjunctival fornix margin for 15 s. The length of the moistened thread was measured in both eyes and the average was used as lacrimal tear volume for comparative analysis.

After the sacrifice, the eyes were collected to analyze the presence of CD45⁺ cells, indicating leukocyte infiltration. All the pictures were quantified with imaged software.

### Results

### Model induction

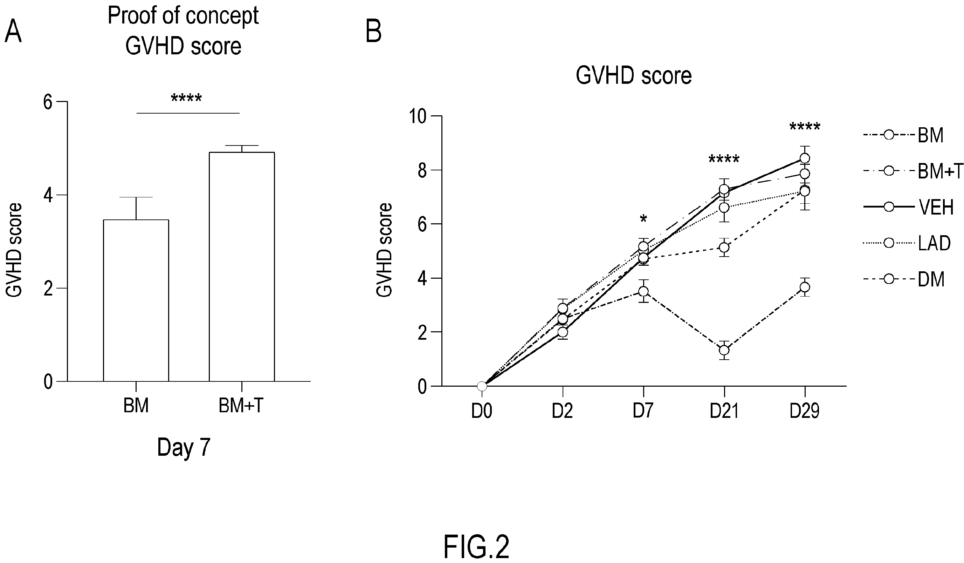

The GVHD model was successfully induced as demonstrated by the GVHD score recorded at different timepoints. Indeed, after 7 days the animals receiving only BM showed a GVHD score statistically milder than animals receiving BM+T cells, indicating the proper time for starting with the treatment (Figure 2A). The following days the positive control (BM group) showed a statistically lower GVHD score compared to other groups, confirming the model induction. Indeed, at day 7, BM had a significantly lower GVHD score compared not only to BM+T but also to VEH and LAD (VEH: *P=0.0392; LAD: **P=0.0062). Similarly, at day 21 and day 29, BM had a significantly lower GVHD score compared to other groups (****P <0.0001). As expected, the BM group showed the variability typical of this model, indeed, at day 21, there was a significant decrease of the GVHD score (**P=0.0018) compared to day 7.

The topical treatment with LAD had no effect on the severity of the systemic GVHD, showing no difference compared to the negative control (BM+T group) (Figure 2B).

### Corneal epitheliopathy (Fluorescein staining)

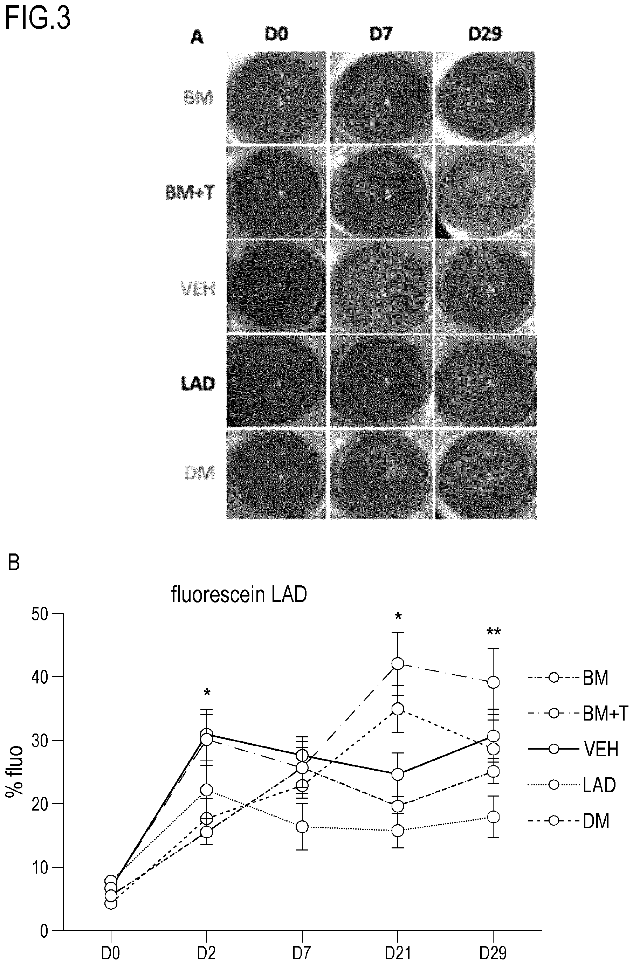

Corneal epitheliopathy was evaluated with fluorescein staining and expressed as a percentage before and after treatment (Figure 3A).

At day 2, before treatment, the BM group developed a significantly milder corneal epitheliopathy compared to BM+T and VEH groups (**P=0.0068 and **P=0.0036, respectively). Also, the DM group had a significantly lower corneal epitheliopathy compared to BM+T and VEH groups (*P=0.0335 and *P=0.0197, respectively).

LAD group showed a significant improvement compared to BM+T at both 21 and 29 days (****P<0.0001 and **P=0.0023, respectively). However, it did not show significant improvement compared to VEH. Moreover, VEH demonstrated a significant decrease compared to BM+T group at day 21 (*P = 0.0108) (Figure 3B). Even though at the starting day of treatment the groups presented a different starting point, the difference was not significant, therefore all the groups can be considered at the same baseline.

Based on the above data, it can be concluded that ladarixin stabilized the corneal epitheliopathy through the treatment and prevented a worsening. Conversely, the corneal epitheliopathy worsened in other groups during the treatment.

### Corneal opacity

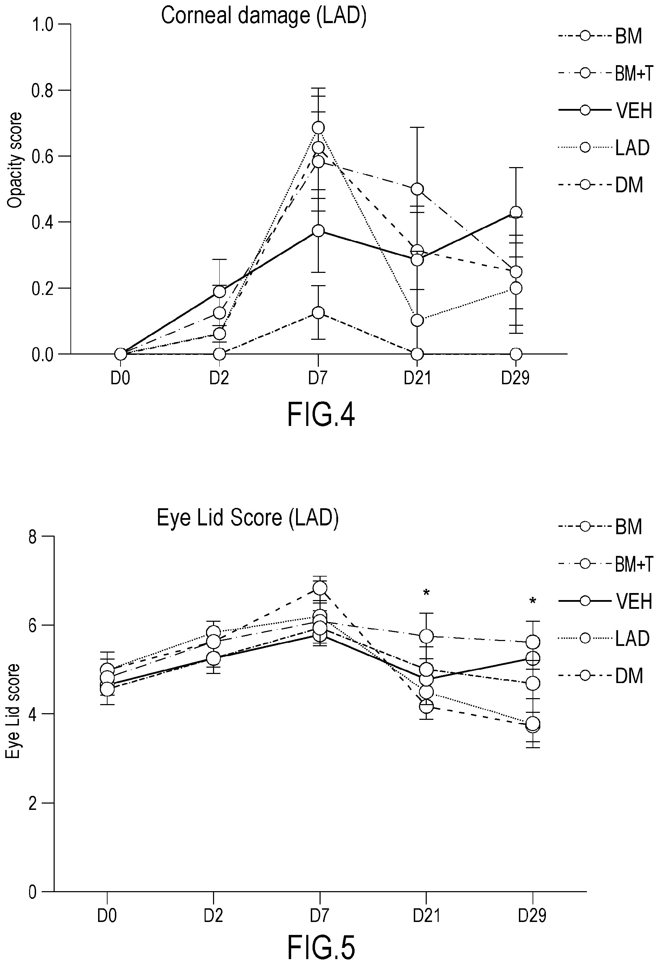

Corneal opacity score was evaluated with light pictures and data were reported in Figure 4.

At day 29, no significant differences were reported among the groups, suggesting that the treatment did not affect corneal opacity (Figure 4).

### Eyelid score

The eyelid score was evaluated with light pictures (Figure 5).

Figure 5 shows that at day 29, LAD and DM had a significantly lower score compared to BM+T (*P=0.0188 and **P=0.0036 respectively). Similarly, LAD and DM had a significantly lower score compared to VEH (*P=0.0347 and

**P=0.0048 respectively). These results suggest an effective improvement of LAD and DM in the eyelid score.

### Leukocyte infiltration (CD45⁺ cell staining)

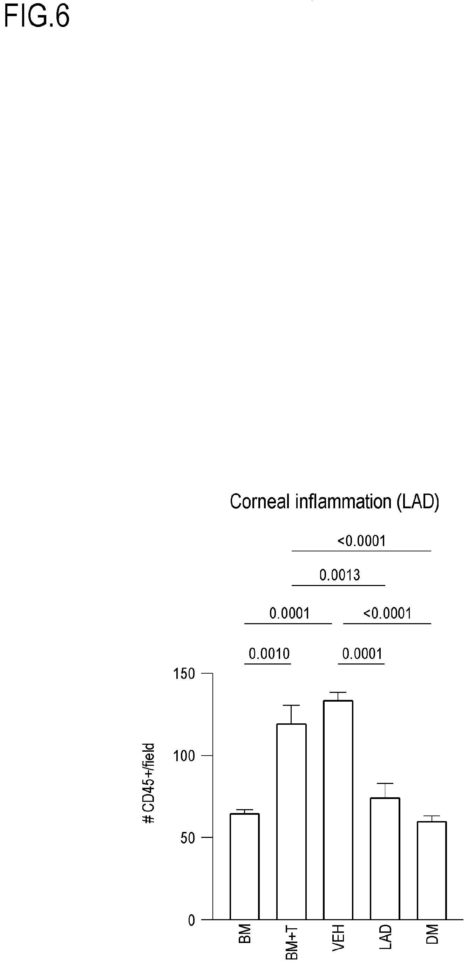

Additionally, markers of leukocyte infiltration indicating inflammation have been considered on whole cornea samples to evaluate the efficacy of the different treatments (Figure 6).

LAD showed a significantly reduced infiltration of CD45⁺ cells compared to VEH and BM+T groups (***P=0.0001 and **P=0.0013 respectively) (Figure 6). As expected, DM showed a significantly reduced leukocyte infiltration compared to VEH and BM+T (****P<0.0001). No significant difference was reported between LAD and DM indicating similar anti-inflammatory effects.

### Conclusions

The following conclusions can be drawn from the above-discussed results:
- The model used effectively recapitulated ocular GVHD pathological changes;
- Corneal epitheliopathy was stabilized by the treatment with ladarixin, thus preventing a worsening of this parameter;
- Corneal opacity was not affected by treatment;
- LAD improved the eyelid score compared to controls and had similar effects to DM;
- LAD reduced leukocyte infiltration compared to VEH and had similar effects as DM.

It is evident from the above conclusions that LAD treatment did not induce adverse reactions and provided beneficial effects on several aspects of the ocular damage caused by GVHD in this model, including both ocular surface and eyelid signs. Interestingly LAD proved better or as effective as DM in the tested endpoints. It can therefore be expected that topical treatment with ladarixin may be effective in reducing cornea, conjunctival and eyelid inflammatory reaction associated with ocular GVHD and their consequences, potentially improving the blepharitis and keratoconjunctivitis caused by oGVHD and the associated ocular discomfort symptoms.

These results demonstrate that the inhibition of CXCL8 exerted by the administration of ladarixin is effective in the treatment of ocular GVHD.

## Claims

1. A CXCL8 inhibitor for use in the treatment of ocular graft-versus-host disease in a subject.

2. A CXCL8 inhibitor for use according to claim 1, wherein said CXCL8 inhibitor is for use in the treatment of ocular inflammation associated with ocular graft-versus-host disease in a subject.

3. A CXCL8 inhibitor for use as claimed in claim 1 or claim 2, wherein said CXCL8 inhibitor is administered topically to the surface of the eye of the subject.

4. A CXCL8 inhibitor for use according to any one of claims 1 to 3, wherein said CXCL8 inhibitor is an anti-CXCL8 antibody or a CXCL8 receptor(s) inhibitor, preferably a CXCR1 inhibitor or a CXCR1/CXCR2 inhibitor.

5. A CXCL8 inhibitor for use according to any one of claims 1 to 4, wherein said CXCL8 inhibitor is an antagonist of the CXCR1 receptor or an antagonist of both CXCR1 and CXCR2 receptors.

6. A CXCL8 inhibitor for use according to any one of claims 1 to 5, wherein said CXCL8 inhibitor is a CXCL8 receptor(s) inhibitor selected from an allosteric inhibitor or an orthosteric antagonist of CXCR1 receptor or of both CXCR1 and CXCR2 receptors.

7. A CXCL8 inhibitor for use according to any one of claims 1 to 6, wherein said CXCL8 inhibitor has general formula (I): 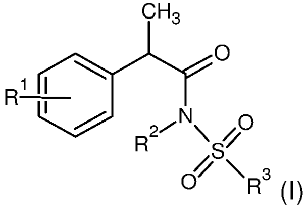 wherein
R¹ is selected from a linear or branched C₁-C₆ alkyl, benzoyl, phenoxy, and trifluoromethanesulfonyloxy;
R² is selected from hydrogen and a linear or branched C₁-C₃ alkyl; and
R³ is a linear or branched C₁-C₆ alkyl or trifluoromethyl,
or a pharmaceutically acceptable salt thereof.

8. A CXCL8 inhibitor for use according to claim 7, wherein the chiral carbon of the compounds of formula (I) is in the R configuration.

9. A CXCL8 inhibitor for use according to any one of claims 1 to 6, wherein said CXCL8 inhibitor has general formula (II): 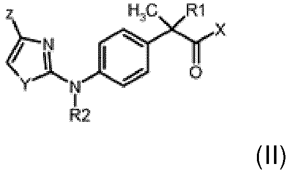 wherein:
R1 is hydrogen or CH₃;
X is OH;
R2 is hydrogen or linear C₁-C₄ alkyl,
Y is a heteroatom selected from S, O and N,
Z is selected from linear or branched C₁-C₄ alkyl, linear or branched C₁-C₄ alkoxy, halo C₁-C₃ alkyl and halo C₁-C₃ alkoxy,
or pharmaceutically acceptable salts thereof.

10. A CXCL8 inhibitor for use according to claim 9, wherein the chiral carbon of the compounds of formula (II) is in the S configuration.

11. A CXCL8 inhibitor for use according to any one of claims 1 to 10, wherein said CXCL8 inhibitor is selected from the group consisting of:
- the anti-CXCL-8 antibodies ABCream, BMS-986253 and ABX-IL-8;
- RP-72, PAC-G-31-P, SCH-N,
- Navarixin, having formula: 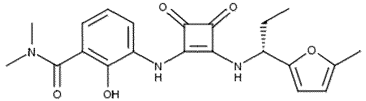
- SX-517, having formula: 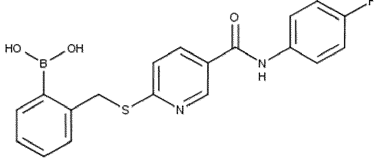
- SX-576, having formula: 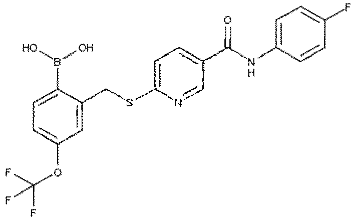
- SX-682, having formula: 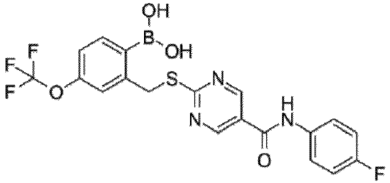
- compounds having formula: 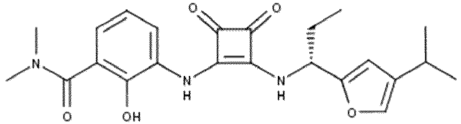 or 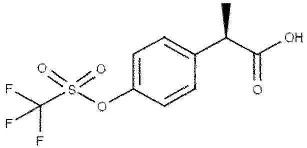 or 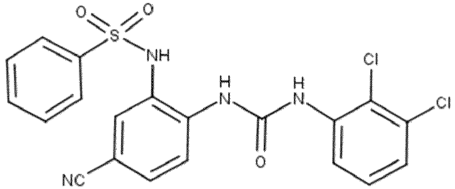 and
- 5-[3-(2-Fluorophenyl)ureido]-1-(2-hydroxypropyl)-1H-pyrazole-4-carboxylic acid ethyl ester
- 5-[3-(3-Fluorophenyl)ureido]-1-(2-hydroxypropyl)-1H-pyrazole-4-carboxylic acid ethyl ester
- 3-[2-[1 (R)-(4-Bromofuran-2-yl)propylamino]-3,4-dioxo-1-cyclobutenylamino]-2-hydroxy-N,N-dimethylbenzamide
- 3-[2-[1(R)-(4-Chlorofuran-2-yl)propylamino]-3,4-dioxo-1-cyclobutenylamino]-2-hydroxy-N,N-dimethylbenzamide
- Trifluoromethanesulfonic acid 4-[1(R)-(N-isopropylcarbamoyl)ethyl]phenyl ester
- 2-Hydroxy-3-[4-[1(R)-(4-isopropylfuran-2-yl)propylamino]-1-oxo-1,2,5-thiadiazol-3-ylamino]-N,N-dimethylbenzamide
- 3-(2-Chlorophenylamino)-7-nitro-4H-1,2,4-benzothiadiazin-5-ol 1,1-dioxide
- 1-[3-[4-[3-(4-Fluorophenyl)isoxazol-5-yl]phenoxy]propyl]-4-methylpiperazine
- N-(2-[(2,3-Difluorobenzyl)sulfanyl]-6-[[(2R,3S)-3,4-dihydroxybutan-2-yl]oxy]pyrimidin-4-yl)azetidine-1-sulfonamide; and
- the compounds of formula (I) and (II) as defined in claims 7 to 10.

12. A CXCL8 inhibitor for use according to claim 7 or 8, wherein said CXCL8 inhibitor has general formula (I) and is selected from:
- 2-(4-isobutylphenyl)propionyl methansulfonamide, preferably R-(-)-2-(4-isobutylphenyl)propionyl methansulfonamide and pharmaceutically acceptable salts thereof, preferably the lysine salt thereof, and
- 2-[(4-trifluoromethanesulfonyloxy)phenyl]-N-methanesulfonyl propionamide, preferably R(-)-2-[(4-trifluoromethanesulfonyloxy)phenyl]-N-methanesulfonyl propionamide and pharmaceutically acceptable salts thereof, in particular the sodium salt thereof.

13. A CXCL8 inhibitor for use according to claim 12, wherein said CXCL8 inhibitor is the sodium salt of R(-)-2-[(4-trifluoromethanesulfonyloxy)phenyl]-N-methanesulfonyl propionamide.

14. A CXCL8 inhibitor for use according to claim 9 or 10, wherein said CXCL8 inhibitor has general formula (II) and is 2-(4-{[4-(trifluoromethyl)-1 ,3-thiazol-2-yl]amino}phenyl)propanoic acid, preferably (2S)-2-(4-{[4-(trifluoromethyl)-1 ,3-thiazol-2-yl] amino} phenyl) propanoic acid or the sodium salt thereof.

15. An ophthalmic composition comprising a CXCL8 inhibitor as defined in any one of claims 1 to 14 and at least one ophthalmologically acceptable excipient or carrier for use in the treatment of ocular graft-versus-host disease in a subject.
